# EUROPEAN PATENT SPECIFICATION

(11) **EP 1 375 674 B1**
(45) Date of publication and mention of the grant of the patent: **16.05.2007**
(21) Application number: 03013424.1
(22) Date of filing: 20.06.2003
(51) Int. Cl.: C12Q 1/68

(54) **Method of detecting nucleic acids using probes with fluorescent labels by analysis of the fluorescence melting curves**
Verfahren zum Nachweis von Nukleinsäuren mit Fluoreszenzfarbstoff-markierten Sonden welches auf der Analyse von Schmelzkurven basiert
Procédé de détection d'acides nucléiques utilisant des sondes avec des marqueurs fluorescents au moyen de courbes de dénaturation thermique

(30) Priority: 21.06.2002 JP 2002181573
(43) Date of publication of application: 02.01.2004
(73) Proprietor: TOSOH CORPORATION, Shunan-shi Yamaguchi-ken 746-8501 (JP)
(72) Inventor: Itoh, Shun-ichi, Sagamihara-shi, Kanagawa-ken (JP); Hayashi, Toshinori, Sagamihara-shi, Kanagawa-ken (JP); Saitoh, Juichi, Yamato-shi, Kanagawa-ken (JP); Ishiguro, Takahiko, Yokohama-shi, Kanagawa-ken (JP)
(74) Representative: Vossius & Partner

(56) References cited:
- EP-A- 1 275 734
- WO-A-00/18965
- WO-A-01/48237
- WO-A-02/14555
- FR-A- 2 750 504
- TAYA T ET AL: "HOMOGENEOUS DETECTION OF A TARGET NUCLEIC ACID SEQUENCE BY COMBINATION OF THE INTERCALATION ACTIVATING FLUORESCENCE DNA PROBE AND THE ISOTHERMAL SEQUENCE AMPLIFICATION" NUCLEIC ACIDS SYMPOSIUM SERIES, IRL PRESS, OXFORD, GB, vol. 42, 1999, pages 51-52, XP001135170 ISSN: 0261-3166
- RIRIE K M ET AL: "PRODUCT DIFFERENTIATION BY ANALYSIS OF DNA MELTING CURVES DURING THE POLYMERASE CHAIN REACTION" ANALYTICAL BIOCHEMISTRY, ACADEMIC PRESS, SAN DIEGO, CA, US, vol. 245, no. 2, 1997, pages 154-160, XP001015991 ISSN: 0003-2697
- ISHIGURO ET AL.: "Fluorescence detection of specific sequence of nucleic acids by oxazole yellow-linked oligonucleotides. Homogeneous quantitative monitoring of in vitro transcription." NUCLEIC ACIDS RESEARCH, vol. 24, no. 24, 1996, pages 4992-4997,

### SEQUENCE LISTING

<110> TOSOH Corporation
<120> METHOD OF DETECTING NUCLEIC ACIDS
<130> PA211-0794
<160> 12
<210> 1
   <211> 48
   <212> DNA
   <213> Artificial Sequence
<220>
   <223> First oligonucleotide (for invA)
<400> 1
   atttctaata cgactcacta tagggagaca ttattatctt tgtgaact 48
<210> 2
   <211> 51
   <212> DNA
   <213> Artificial Sequence
<220>
   <223> First oligonucleotide (for VT2)
<400> 2
   aattctaata cgactcacta tagggagatt ctaccgtttt tcagatttta c 51
<210> 3
   <211> 51
   <212> DNA
   <213> Artificial Sequence
<220>
   <223> First oligonucleotide (for tdh2)
<400> 3
   aattctaata cgactcacta tagggagaac aacttttaat accaatgcac c 51
<210> 4
   <211> 18
   <212> DNA
   <213> Artificial Sequence
<220>
   <223> Second oligonucleotide (for invA)
<400> 4
   atcaacaatg cggggatc 18
<210> 5
   <211> 20
   <212> DNA
   <213> Artificial Sequence
<220>
   <223 Second oligonucleotide (for VT2)
<400> 5
   gtaaggcttc tgctgtgaca 20
<210> 6
   <211> 20
   <212> DNA
   <213> Artificial Sequence
<220>
   <223 Second oligonucleotide (for tdh2)
<400> 6
   attaccaata tattaccact 20
<210> 7
   <211> 20
   <212> DNA
   <213> Artificial Sequence
<220>
   <223> Third oligonucleotide (for invA)
<400> 7
   taatgatgcc ggcaatagcg 20
<210> 8
   <211> 39
   <212> DNA
   <213> Artificial Sequence
<220>
   <223> Third oligonucleotide (for VT2)
<400> 8
   tagaaagtat ttgttgccgt attaacgaac ccggccaca 39
<210> 9
   <211> 10
   <212> DNA
   <213> Artificial Sequence
<220>
   <223> Third oligonucleotide (for tdh2)
<400> 9
   gttgtatctc gaacaacaaa 20
<210> 10
   <211> 26
   <212> DNA
   <213> Artificial Sequence
<220>
   <223> Intercalator-labeled probe (for invA)
<400> 10
   tcagcatggt ataagtagac agggcg 26
<210> 11
   <211> 10
   <212> DNA
   <213> Artificial Sequence
<220>
   <223> Intercalator-labeled probe (for VT2)
<400> 11
   ttaacgccag atatgatgaa 20
<210> 12
   <211> 10
   <212> DNA
   <213> Artificial Sequence
<220>
   <223> Intercalator-labeled probe (for tdh2)
<400> 12
   acgtgtactt gatctgattt 20

### SEQUENCE LISTING

<110> TOSOH Corporation
<120> METHOD OF DETECTING NUCLEIC ACIDS
<130> PA 211-0794
<160> 12
<170> PatentIn version 3.1
<210> 1
   <211> 48
   <212> DNA
   <213> Artificial
<220>
   <223> First oligonucleotide (for invA), designed based on the RNA seque nce of Salumonella spp.
<400> 1
   atttctaata cgactcacta tagggagaca ttattatctt tgtgaact 48
<210> 2
   <211> 51
   <212> DNA
   <213> Artificial
<220>
   <223> First oligonucleotide (for VT2), designed based on the RNA sequen ce of E. Coli O-157.
<400> 2
   aattctaata cgactcacta tagggagatt ctaccgtttt tcagatttta c 51
<210> 3
   <211> 51
   <212> DNA
   <213> Artificial
<220>
   <223> First oligonucleotide (for tdh2), designed based on the RNA seque nce of Vibrio parahaemolyticus.
<400> 3
   aattctaata cgactcacta tagggagaac aacttttaat accaatgcac c 51
<210> 4
   <211> 18
   <212> DNA
   <213> Artificial
<220>
   <223> Second oligonucleotide (for invA), designed based on the RNA sequ ence of Salumonella spp.
<400> 4
   atcaacaatg cggggatc 18
<210> 5
   <211> 20
   <212> DNA
   <213> Artificial
<220>
   <223> Second oligonucleotide (for VT2), designed based on the RNA seque nce of E. Coli O-157.
<400> 5
   gtaaggcttc tgctgtgaca 20
<210> 6
   <211> 20
   <212> DNA
   <213> Artificial
<220>
   <223> Second oligonucleotide (for tdh2), designed based on the RNA sequ ence of Vibrio parahaemolyticus.
<400> 6
   attaccaata tattaccact 20
<210> 7
   <211> 20
   <212> DNA
   <213> Artificial
<220>
   <223> Third oligonucleotide (for invA), designed based on the RNA seque nce of Salumonella spp.
<400> 7
   taatgatgcc ggcaatagcg 20
<210> 8
   <211> 39
   <212> DNA
   <213> Artificial
<220>
   <223> Third oligonucleotide (for VT2), designed based on the RNA sequen ce of E. Coli O-157.
<400> 8
   tagaaagtat ttgttgccgt attaacgaac ccggccaca 39
<210> 9
   <211> 20
   <212> DNA
   <213> Artificial
<220>
   <223> Third oligonucleotide (for tdh2), designed based on the RNA seque nce of Vibrio parahaemolyticus.
<400> 9
   gttgtatctc gaacaacaaa 20
<210> 10
   <211> 26
   <212> DNA
   <213> Artificial
<220>
   <223> Intercalator-labeled probe (for invA), designed based on the RNA sequence of Salumonella spp.
<400> 10
   tcagcatggt ataagtagac agggcg 26
<210> 11
   <211> 20
   <212> DNA
   <213> Artificial
<220>
   <223> Intercalator-labeled probe (for VT2), designed based on the RNA s equence of E. Coli O-157.
<400> 11
   ttaacgccag atatgatgaa 20
<210> 12
   <211> 20
   <212> DNA
   <213> Artificial
<220>
   <223> Intercalator-labeled probe (for tdh2), designed based on the RNA sequence of Vibrio parahaemolyticus.
<400> 12
   acgtgtactt gatctgattt 20

## Description

The present invention relates to analysis of specific nucleic acid sequences anticipated in gene mixtures containing DNA or RNA and is useful for gene diagnosis in the field of clinical diagnosis.

For high sensitivity detection of nucleic acids, amplification of target nucleic acids can be utilized. As a technique for amplifying specific DNA sequences, polymerase chain reaction (PCR) is known. The need for rapid heating and cooling in PCR is the major barrier to shorter reaction time and automation of PCR. On the other hand, as techniques for isothermal nucleic acid amplification, NASBA and 3SR are known to amplify specific RNA sequences by the cooperative action of a reverse transcriptase and an RNA polymerase. These techniques involve the chain reaction comprising synthesis of a double-stranded DNA having a promoter sequence from the target RNA as the template by using a primer having a promoter sequence, a reverse transcriptase and ribonuclease H, and synthesis by an RNA polymerase of an RNA having the specific base sequence in the target RNA, which is then used as the template for synthesis of the above-mentioned double-stranded DNA having the promoter sequence. They afford isothermal amplification and are suitable for continuous processing and automation.

In any cases, the amplification product is usually detected through separation analysis such as electrophoresis or known nucleic acid detection methods based on hybridization methods. Especially, hybridization methods using an oligonucleotide probe labeled with a substance which gives off a detectable signal are used for selective detection of the amplification product.

In hybridization methods, the nucleic acid obtained as the PCR or NASBA amplification product is usually brought into contact with the oligonucleotide probe after separation from the reaction solution. In hybridization methods using an oligonucleotide probe labeled with an fluorescent intercalative dye (hereinafter sometimes referred to as an intercalator-labeled probe), PCR or NASBA can be carried out in the presence of the oligonucleotide probe while the change in the fluorescence of the reaction solution is measured. The intercalator-labeled probe is an oligonucleotide having a fluorescent intercalative dye linked to a phosphorus atom in it via a linker. It alters its fluorescence when it forms a complementary duplex with the target nucleic acid by intercalating the intercalator moiety into the duplex. This enables the target nucleic acid to be detected without separation analysis (Ishiguro, et al., (1996) Nucleic Acids Res. 24(24) 4992-4997). Thus, the combination of a fluorescent intercalative dye-labeled probe and the above-mentioned techniques for amplifying specific RNA sequences makes it possible to measure the amplification product selectively without separation analysis of the amplification product or heating and cooling.

The combination of an intercalator-labeled probe and the above-mentioned amplification of specific RNA sequences provides an assay system suitable for automation. However, there are problems which have to be solved to detect two or more nucleic acids simultaneously. In other words, there are problems of the difficulty in selecting fluorescent intercalative dyes which are detectable distinguishably at the same time from a great variety of fluorescent intercalative dyes available as the label and the need for multiwavelength fluorescence measurements, in some cases, at more than one excitation wavelengths which limits the availability of detectors, if any, to complex and expensive ones. Besides, in the case of simultaneous detection of at least three nucleic acids, another problem arises because it takes a long time to prepare at least three intercalator-labeled probes by linking preliminarily chosen at least three mutually distinguishable fluorescent intercalative dyes to oligonucleotides. The lack of quick adaptability to an increase of nucleic acids to be detected is also problematic in actual operations.

There are some ways to obviate the above-mentioned problems with one fluorescent intercalative dye. For example, it is possible to detect two or more nucleic acids with a single fluorescent intercalative dye by preparing as many portions (vessels) of a reaction solution as the nucleic acids to detect and add intercalator-labeled probes having oligonucleotides complementary to the nucleic acids to be detected. However, in this case, if two or more nucleic acids in a sample are to be detected, the need to prepare at least as many portions of a reaction solution as the nucleic acids to be detected creates problems from the aspects of cost and the like such as high demand on the throughput capacity of the amplification and detection instruments. Further, the need to prepare two or more reaction solutions leads to an increase in the amount of a sample to be used for the detection.

WO 00/18965 discloses a method of detecting two nucleic acids in a sample (i.e. at positions corresponding to C282Y or H63D in the hemochromatosis genes) (example 8, Fig. 20), which comprises (1) adding to the sample plural nucleic acid probes complementary to the respective nucleic acids which are labelled with the same fluorescent substance (2) a step of carrying out nucleic acid amplification (multiplexed PCR reactions comprising two primer pairs i.e. primers of SEQ ID 16-18), and (3) detecting the respective nucleic acids in one vessel simultaneously on the basis of the different Tm values of the duplexes.

Therefore, there is a demand for a detection method which does not encounter the above-mentioned problems even if only one fluorescent intercalative dye is used.

The present invention provides a method of detecting at least two target nucleic acids in one vessel at a single fluorescent wavelength simultaneously. Namely, the invention as defined in Claim 1 provides a method of detecting at least two nucleic acids in a sample, which comprises (1) a step of adding to the sample plural nucleic acid probes complementary to the respective nucleic acids which are labeled with the same fluorescent substance, (2) a step of carrying out isothermal nucleic acid amplification under conditions that allow a chain reaction comprising synthesis of double-stranded DNAs having promoter sequences at the end using the nucleic acids as the templates, transcription of the double-stranded DNAs into RNA transcripts and synthesis of the double-stranded DNAs using the resulting RNA transcripts, (3) allowing the resulting RNA transcripts to form duplexes with said plural nucleic acid probes, and (4) detecting the resulting RNA transcripts in one vessel simultaneously on the basis of the different Tm values of said duplexes, wherein said plural nucleic acid probes are designed so as to alter their fluorescence intensities when they form duplexes with the resulting transcripts.

The invention as defined in Claim 2 provides the method of detecting at least two nucleic acids in a sample according to Claim 1, which comprises (1) a step of measuring the fluorescent intensity while changing the temperature of the sample continuously, and (2) a step of simultaneously detecting the resulting RNA transcripts by determining the Tm values from the resulting fluorescence profile.

The invention as defined in Claim 3 provides the invention according to Claim 1 or 2, wherein said plural nucleic acid probes are prepared by linking a fluorescent intercalative dye to oligonucleotides via linkers.
Fig. 1 shows the fluorescent melting curves obtained by changing the temperatures of the RNAs obtained by amplifying the respective standard RNAs separately (in the presence of intercalator-labeled probes).
Fig. 2 is the negative first derivatives of the melting curves shown in Fig. 1 wherein the peaks (Tm values) of the negative first derivative of the fluorescence intensity (-dF/dT) are indicated.
Fig. 3 is the negative first derivative of the fluorescent melting curve obtained by changing the temperature of the amplification products obtained by simultaneously amplifying the three standard RNAs in one tube wherein the peaks (Tm values) of the negative first derivative of the fluorescence intensity (-dF/dT) are indicated.

Now, the present invention will be described in detail.

The present invention provides a method of detecting at least two nucleic acids to be detected (hereinafter referred to as target nucleic acids) simultaneously as referred to in the claims, specifically a method comprising adding intercalator-labeled probes complementary to the target nucleic acids which form duplexes having different Tm values and have the same fluorescent dye, preferably the same fluorescent intercalative dye as the label to a single reaction solution (in a single vessel) and measuring the fluorescence intensity while changing the temperature.

It is generally known that hybrid double strands of nucleic acid denature into single strands when heated in a solution. The temperature at which 50% of the double strands of nucleic acid dissociate into single strands is called Tm and indicates the stability of the double-stranded nucleic acid. Because Tm is known to depend on the length of the complementary sequence in the hybrid and the content of guanine (G) and cytosine (C) in the sequence, it is possible to tailor arbitrary probes which form duplexes having different Tm values for use in the present invention.

The probes are preferably prepared so as to secure large differences in Tm values. In the present invention, "large differences" means that the respective Tm values are distinguishable from each other when the temperature is changed in the same manner at the same heating rate (the rate at which the temperature rises per a unit time) as those employed in the present invention. In general, the combination of a highly sensitive detector and a continuous slow temperature change over a long time is enough to detect such small Tm differences as about 3°C as in the after-mentioned Examples. On the contrary, if a low-sensitive detector is used, it is necessary to change the temperature intermittently (for example, 5°C at a time) or use probes which form duplexes having widely different Tm values while changing the temperature in a short time.

In the present invention, the target nucleic acids may be single-stranded or double-stranded RNAs or DNAs. For example, as single-stranded RNAs, mRNA, tRNA, rRNA and the genomic RNA of an RNA virus may be mentioned. In the case of a single-stranded DNA, it is possible to insert a single-stranded DNA into M13 vector, propagate the phage and recover it from the propagated phage particles by extracting nucleic acid. When a target nucleic acid is a double-stranded nucleic acid, it is necessary to dissociate it into single strands by heat denaturation or alkaline denaturation, and then, if necessary, separate the single strands. It is disclosed herein to amplify a target nucleic acid by techniques for amplifying specific RNA sequences such as NASBA and 3SR and use the resulting single-stranded RNA. The amplification of specific RNA sequences by these techniques is carried out under such conditions that a pair of primers at least one of which has a promoter sequence, a reverse transcriptase and an RNA polymerase (if necessary, and ribonuclease H) can function and uses a chain reaction comprising synthesis of a double-stranded DNA containing a promoter sequence using the target nucleic acid as the template, synthesis of an RNA containing a specific RNA sequence using the double-stranded DNA as the template by the RNA polymerase and subsequent synthesis of a double-stranded DNA containing the promoter sequence using the RNA as the template. The double-stranded DNA containing the promoter sequence is obtained not only through DNA synthesis from an RNA as the target nucleic acid by a reverse transcriptase but also by using known genetic engineering techniques. The RNA amplification product can be used for the subsequent Tm measurement without modifications.

The probes used in the present invention are labeled with the same fluorescent substance, so as to alter the fluorescence intensities when they form duplexes with the target nucleic acids and exemplified by intercalator-labeled probes obtained by linking fluorescent intercalative dyes to oligonucleotides via linkers. Since in the presence of probes labeled so as to alter the fluorescent intensities upon formation of duplexes with the target nucleic acids, different fluorescent signals are detected when the probes are hybridized with the target nucleic acids and when they are unhybridized, separation of the probe molecules hybridized with the target nucleic acid from the unhybridized probe molecules is unnecessary, and it is possible to measure the Tm values while changing the temperature. On the other hand, it is disclosed herein, when the probes are not so designed as to alter the fluorescence intensities upon formation of duplexes with the target nucleic acid, detection may be preceded by separation of either (or both) of the probe molecules hybridized and unhybridized with the target nucleic acid at constant temperatures. The separation procedure may comprise, for example, trapping the target nucleic acids on a solid phase via oligonucleotides immobilized on the solid phase and hybridizing the probe with the trapped target nucleic acids and removing the liquid phase. Now, the present invention will be described in reference to embodiments using intercalator-labeled probes.

When the above-mentioned intercalator-labeled probes and the above-mentioned RNA amplification are used, the intercalator-labeled probes are added in advance to the RNA amplification reaction solution and carry out RNA amplification while measuring the fluorescence intensity to monitor the amount of the amplification product. Subsequently, the RNA amplification product is gradually heated, gradually cooled after preliminary heat denaturation, or gradually heated after preliminary heat denaturation and subsequent quenching, under temperature control to obtain the melting curve of the nucleic acids.

The Tm value is obtained at the midpoint between the inflection points of the melting curve or at the maximum point (in terms of absolute value) of the first derivatives of the melting curves. Comparison between the measured Tm values and the intended Tm values of the duplexes resulting from the probes indicates whether the observed fluorescent enhancements are attributed to the presence of the amplification products from the specific RNAs.

The above-mentioned procedure allows plural nucleic acids to be detected in one vessel simultaneously by distinguishing the Tm values using plural probes labeled with the same fluorescent intercalative dye. In other words, the above-mentioned amplification of specific RNA is carried out under conditions which allow amplification of at least two target nucleic acids, and nucleic acid probes complementary to the respective amplification products which form duplexes having different Tm values with the respective amplification products and are labeled with the same fluorescent intercalative dye are added before the amplification reaction. Then, the temperature is changed as explained above while the fluorescence intensity is measured with time. Thus, a melting curve is obtained, and by analyzing the melting curve as described above, it is possible to detect whether the target nucleic acids are present by the peaks in the first derivative of the melting curve at the Tm values intended for the duplexes resulting from the respective probes. Any fluorometers that can measure fluorescence intensity under temperature control or monitoring such as LightCycler Quick System 330 (Roche Diagnostics Co., Ltd.) are available for the method of the present invention without any particular restrictions.

Now, the present invention will be described in further detail in reference to Examples. However, the present invention is by no means restricted to these specific Examples.

### Example 1 Amplification of target RNA and measurement of Tm values

(1) As standard RNAs, invA (a 1954-nt RNA containing the sequence of an invasion-associated gene in Salumonella spp.), VT2 (a 1361-nt RNA containing the sequence of the verotoxin type 2 gene in E. coli O-157) and tdh2 (a 616-nt RNA containing the sequence of the thermostable hemolysin gene in Vibrio paraharmolyticus) were used.
   The standard RNAs having these sequences were diluted with an RNA diluent (10 mM Tris-HCl (pH 8.0), 0.1 mM EDTA, 1mM DTT, 0.5 U/µl RNase inhibitor) to 10000 copies/10 µl, respectively, for use as RNA samples.
(2) The reaction solution for amplification of invA, VT2 and tdh2 having the following composition was dispensed into 0.5 ml PCR tubes (GeneAmp Thin-Walled Reaction Tubes, Perkin-Elmer) (20 µl each), and 5 µl of the RNA samples were added. The sequences of the first oligonucleotide (sense primer containing the T7 RNA polymerase promoter sequence), the second oligonucleotide (antisense primer), the third oligonucleotide (scissor oligonucleotide having a 3' end modified with an amino group (NH₂) for cleavage of the standard RNAs) and intercalator-labeled probes were SEQ ID NOS:1, 4, 7 and 10 for invA, SEQ ID NOS:2, 5, 8 and 11 for VT2 and SEQ ID NOS:3, 6, 9 and 12 for tdh2.
   The composition of the reaction solution (concentrations in the final volume of 30 µl)
   60 mM Tris-HCl buffer (pH 8.0)
   18.7 mM Magnesium chloride
   120 mM Potassium chloride
   6 U RNase Inhibitor
   1 mM DTT
   0.25 mM each of dATP, dCTP, dGTP and dTTP
   3.6 mM ITP
   3.0 mM each of Atp, CTP, GTP and UTP
   1.0 µM first oligonucleotides (SEQ ID NOS:1, 2 and 3)
   1.0 µM second oligonucleotides (SEQ ID NOS:4, 5 and 6)
   0.16 µM third oligonucleotides (SEQ ID NOS:7, 8 and 9)
   13% DMSO
   25 nM intercalator-labeled probes (SEQ ID NOS:10, 11 and 12)
   Distilled water for volume adjustment
(3) The intercalator-labeled probes complementary to the respective standard RNAs were prepared by linking oxazole yellow as a fluorescent intercalative dye, to the phosphorus atom in the 13rd "A" for the one used for invA (SEQ ID NO:10), to the phosphorus atom in the 12th "T" for the one used for VT2 (SEQ ID NO:11) and to the phosphorus atom in the 16th "G" for the one used for tdh2 (SEQ ID NO:12) via linkers according to Ishiguro et al. (Nucleic Acids Res., 24(24) 4992-4997 (1996)).
(4) The resulting reaction mixtures were incubated at 41°C for 5 minutes, and 5 µl of an enzyme solution of the following composition was added.
   The composition of the enzyme solution (concentrations in the final volume of 30 µl)
   1.7% Sorbitol
   3.6 µg Bovine serum albumin
   142 U T7 RNA polymerase (Gibco)
   8 U AMV reverse transcriptase (Takara Shuzo Co., Ltd.)
   1 U/ml RNase H
   Distilled water for volume adjustment
(5) Then, the PCR tubes were incubated at 41°C for 30 minutes to amplify the RNAs.
(6) The solutions were set in a fluorescent spectrometer with a thermostatic sample chamber, and the fluorescent intensity was monitored (at an excitation wavelength of 470 nm and an emission wavelength of 520 nm) while the reaction solutions were heated at 95°C for 1 second, then cooled to 41°C and heated from 41°C to 85°C at a rate of 0.1 °C/second. The Tm values were obtained from the fluorescence profile (the melting curve).

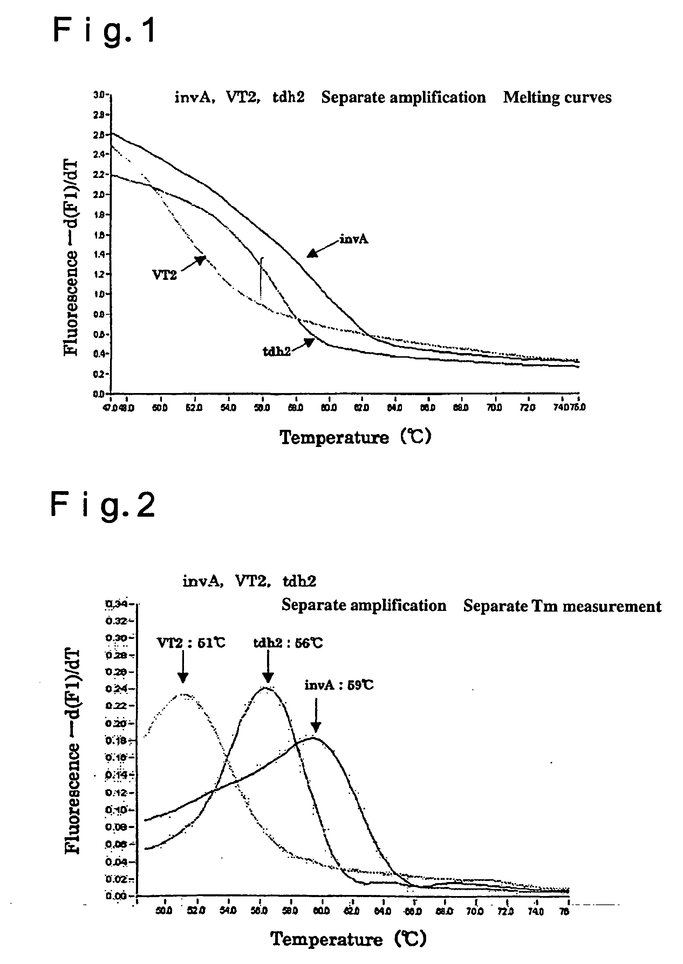

(7) Fig. 1 shows the melting curves for the respective standard RNAs, and Fig. 2 shows first derivatives of the melting curves. The first derivatives of the melting curves for all the three amplification products (including the probes) showed peaks at specific Tm values with no overlap. The distinguishably detected Tm values demonstrate the presence of the respective amplification products of these genes and the standard RNAs used as the templates in the amplification. Example 2 Simultaneous amplification and detection of three target RNAs

(1) The same standard RNAs (invA, VT2 and tdh2) as used in Example 1 were used. The standard RNAs having the previously mentioned sequences were diluted with an RNA diluent (10 mM Tris-HCl (pH 8.0), 0.1 mM EDTA, 1 mM DTT, 0.5 U/µl RNase Inhibitor) to 10000 copies/10 µl for use as RNA samples.
(2) 20 µl of a reaction solution having the following composition (for simultaneous amplification of invA, VT2 and tdh2) was dispensed into 0.5 ml PCR tubes (GeneAmp Thin-Walled Reaction Tubes, Perkin-Elmer), and 5 µl of the RNA samples were added.
   The composition of the reaction solution (concentrations in the final volume of 30 µl)
   60 mM Tris-HCl buffer (pH 8.0)
   18.7 mM Magnesium chloride
   120 mM Potassium chloride
   6 U RNase Inhibitor
   1 mM DTT
   0.25 mM each of dATP, dCTP, dGTP and dTTP
   3.6 mM ITP .
   3.0 mM each of Atp, CTP, GTP and UTP
   1.0 µM first oligonucleotides (the same as used in Example 1)
   1.0 µM second oligonucleotides (the same as used in Example 1)
   0.16 µM third oligonucleotides (the same as used in Example 1)
   13% DMSO
   25 nM intercalator-labeled probes (the same as used in Example 1)
   Distilled water for volume adjustment
(3) The resulting reaction mixtures were incubated at 41°C for 5 minutes, and 5 µl of an enzyme solution of the following composition was added.
   The composition of the enzyme solution (concentrations in the final volume of 30 µl)
   1.7% Sorbitol
   3.6 µg Bovine serum albumin
   142 U T7 RNA polymerase
   8 U AMV reverse transcriptase (Takara Shuzo Co., Ltd.)
   1 U/ml RNase H
   Distilled water for volume adjustment
(4) Then, the PCR tubes were incubated at 41°C for 30 minutes for RNA amplification.
(5) The solutions were set in a fluorescent spectrometer with a thermostatic sample chamber, and the fluorescence intensity was monitored (at an excitation wavelength of 470 nm and an emission wavelength of 520 nm) while the reaction solutions were heated at 95°C for 1 second, then cooled to 41°C and heated from 41°C to 85°C at a rate of 0/1 °C/second. The Tm values were obtained from the fluorescent profile (the melting curve).

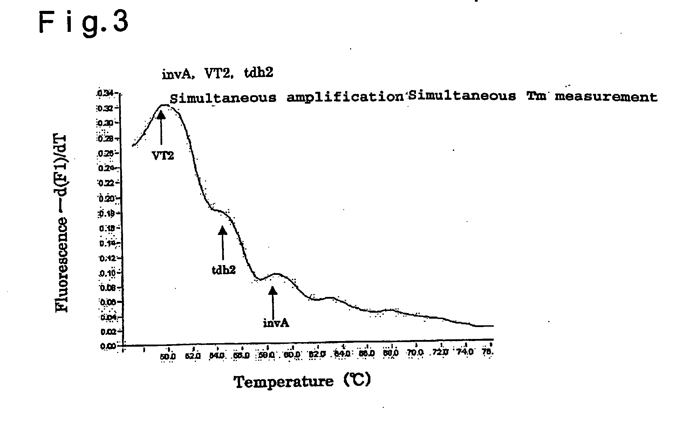

Fig. 3 shows the negative first derivative of the melting curves for the RNAs obtained by simultaneous amplification of the three standard RNAs, and Table 1 shows the Tm values obtained from the results of Example 1 and Example 2 (Figs. 2 and 3). The three products of the simultaneous amplification could be distinguished by the Tm values at which the derivative of the curve has peaks. Thus, it is demonstrated that the present invention enables simultaneous detection of multiple items in one vessel with nucleic acid probes labeled with the same fluorescent intercalative dye. The distinguishably detected Tm values demonstrates the presence of the respective gene amplification products from these genes and the standard RNAs used as the templates in the amplification.

**Table 1**

| Tm values obtained by separate amplification and simultaneous amplification | | |
|---|---|---|
| Nucleic acid | Separate amplification | Simultaneous amplification |
| invA | 59 | 59 |
| VT2 | 51 | 51 |
| tdh2 | 56 | 54 |

As discussed above, the present invention makes it possible to detect at least two nucleic acids simultaneously with a single fluorescent dye label. Consequently, there is no need to prepare plural oligonucleotide probes having fluorescent dyes selected so as to be mutually distinguishable and solves the problem of time-consuming probe preparation and the lack of quick adaptability to an increase of the nucleic acids to be detected in actual operations.

In the present invention, target nucleic acids are detected on the basis of their Tm values. Therefore, despite of the use of a single fluorescent dye as the label, there is no need to prepare as many portions of a reaction solution as the target nucleic acids, and the present invention does not have the problems of the demand for high throughput amplification and detection instruments and the need to increase the amount of samples used for the detection.

When a fluorescent intercalative dye is used in the present invention, the need to separate the probe hybridized with the target nucleic acids and the unhybridized probe for determination of Tm values is obviated. Therefore, the embodiment of the present invention using intercalator-labeled probes is preferable to provide a measuring method which is easy to automate, that is, it enables simultaneous detection of plural target nucleic acids without the need of plural thermoregulators or (optical) detectors for plural portions of a reaction solution. Thus, the present invention is useful in simplifying the instrument, reducing the costs and automation.

## Claims

1. A method of detecting at least two nucleic acids in a sample, which comprises
(1) a step of adding to the sample plural nucleic acid probes complementary to the respective nucleic acids which are labeled with the same fluorescent substance,
(2) a step of carrying out isothermal nucleic acid amplification under conditions that allow a chain reaction comprising synthesis of double-stranded DNAs having promoter sequences at the end using the nucleic acids as the templates, transcription of the double-stranded DNAs into RNA transcripts and synthesis of the double-stranded DNAs using the resulting RNA transcripts,
(3) allowing the resulting RNA transcripts to form duplexes with said plural nucleic acid probes, and
(4) detecting the resulting RNA transcripts in one vessel simultaneously on the basis of the different Tm values of said duplexes,
wherein said plural nucleic acid probes are designed so as to alter their fluorescence intensities when they form duplexes with the resulting transcripts.

2. The method according to claim 1, which comprises
(1) a step of measuring the fluorescent intensity while changing the temperature of the sample continuously, and
(2) a step of simultaneously detecting the resulting RNA transcripts by determining the Tm values from the resulting fluorescence profile.

3. The method according to claim 1 or 2, wherein said plural nucleic acid probes are prepared by linking a fluorescent intercalative dye to oligonucleotides via linkers.

## Patentansprüche

1. Verfahren zum Nachweis mindestens zweier Nucleinsäuren in einer Probe, das umfasst
(1) einen Schritt des Hinzufügens mehrer Nucleinsäuresonden, die komplementär zu den entsprechenden Nucleinsäuren sind und die mit der gleichen fluoreszierenden Substanz markiert sind, zu der Probe,
(2) einen Schritt des Durchführens isothermischer Nucleinsäureamplifikation unter Bedingungen, die eine Kettenreaktion erlauben, die die Synthese von doppelsträngigen DNAs mit Promotorsequenzen am Ende unter Verwendung der Nucleinsäuren als Matrizen, die Transkription der doppelsträngigen DNAs in RNA-Transkripte und die Synthese der doppelsträngigen DNAs unter Verwendung der sich ergebenden RNA-Transkripte umfasst,
(3) Ermöglichen, dass die sich ergebenden RNA-Transkripte mit den mehreren Nucleinsäuresonden Duplexe ausbilden, und
(4) das gleichzeitige Nachweisen der sich ergebenden RNA-Transkripte in einem Gefäß, auf der Basis der unterschiedlichen Tm-Werte der Duplexe,
wobei die mehreren Nucleinsäuresonden so gestaltet sind, dass sie ihre Fluoreszenzintensitäten ändern, wenn sie Duplexe mit den sich ergebenden Transkripten ausbilden.

2. Verfahren gemäß Anspruch 1, umfassend
(1) einen Schritt des Messens der Fluoreszenzintensität, während die Temperatur der Probe kontinuierlich geändert wird, und
(2) einen Schritt des gleichzeitigen Nachweisens der sich ergebenden RNA-Transkripte durch das Ermitteln der Tm-Werte aus dem sich ergebenden Fluoreszenzprofil.

3. Verfahren gemäß Anspruch 1 oder 2, wobei die mehreren Nucleinsäuresonden durch Verknüpfen eines fluoreszierenden interkalierenden Farbstoffs über Linker an Oligonucleotide hergestellt werden.

## Revendications

1. Procédé pour détecter au moins deux acides nucléiques dans un échantillon, qui comprend
(1) une étape d'addition à l'échantillon de plusieurs sondes d'acides nucléiques complémentaires des acides nucléiques respectifs qui sont marquées avec la même substance fluorescente,
(2) une étape de mise en oeuvre d'une amplification d'acides nucléiques isotherme dans des conditions qui permettent une réaction en chaîne comprenant la synthèse d'ADNs double brin ayant des séquences de promoteur à l'extrémité, en utilisant les acides nucléiques en tant que matrices, la transcription des ADNs double brin en produits de transcription d'ARN et la synthèse des ADN double brin en utilisant les produits de transcription d'ARN résultants,
(3) l'opération consistant à permettre aux produits de transcription d'ARN résultants de former des duplex avec lesdites sondes d'acides nucléiques, et
(4) la détection des produits de transcription d'ARN résultants dans un seul récipient simultanément sur la base des différentes valeurs Tm desdits duplex,
dans lequel lesdites sondes d'acides nucléiques sont conçues de façon à altérer leurs intensités de fluorescence quand elles forment des duplex avec les produits de transcription résultants.

2. Procédé selon la revendication 1, qui comprend
(1) une étape de mesure de l'intensité de fluorescence tout en changeant la température de l'échantillon en continu, et
(2) une étape de détection simultanée des produits de transcription d'ARN résultants par détermination des valeurs Tm à partir du profil de fluorescence résultant.

3. Procédé selon la revendication 1 ou 2, dans lequel lesdites sondes d'acides nucléiques sont préparées par liaison d'un colorant intercalant fluorescent à des oligonucléotides via des lieurs.
